# EUROPEAN PATENT APPLICATION

(11) **EP 4 516 784 A1**
(43) Date of publication of application: **05.03.2025**
(21) Application number: 23194584.1
(22) Date of filing: 31.08.2023
(51) Int. Cl.: C07D 317/30, C07D 319/06, C07D 321/06, C07D 405/04, C07D 493/04, C11D 1/08

(54) **SURFACE ACTIVE ALDARIC ACID DERIVATIVES**

(71) Applicant: Coöperatie Koninklijke Cosun U.A., 4814 NE Breda (NL)
(72) Inventor: RANOUX, Adeline, 4671 VA Dinteloord (NL); RAAIJMAKERS, Henricus Wilhelmus Carolina, 4671 VA Dinteloord (NL); VAN DER KLIS, Frits, 6708 WG Wageningen (NL); VAN ES, Daniel Stephan, 6708 WG Wageningen (NL); FAIRGRIEVE, Craig Jonathon, Bebington, CH63 3JW (GB); GRAINGER, David Stephen, Bebington, CH63 3JW (GB)
(74) Representative: Nederlandsch Octrooibureau

(57) **Abstract**

The present invention relates to novel surface active compounds. In a first particular aspect, the invention relates to aldaric acid derivatives having a structure represented by formula (I). The incorporation of one or more aldaric acid derivatives according to the invention in laundry compositions enhances the performance thereof, in particular the ability to remove particulate-soil. In a second aspect, the invention provides compositions comprising one or more aldaric acid derivatives according to formula (I). In a third aspect, a method is provided for preparing the aldaric acid derivatives according to formula (I).

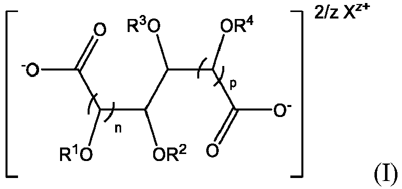

## Description

### FIELD OF THE INVENTION

The present invention relates to novel surface active compounds and compositions thereof. The invention further relates to methods for their production.

### BACKGROUND OF THE INVENTION

Surface active substances, also called surfactants, are important components in a wide variety of home- and personal care products, as well as professional cleaning products. Surfactants can fulfill a multitude of roles such as detergent, wetting agent, emulsifier, foaming agent, dispersant, flotation agent, etc.

A typical surface active compound consists of a hydrophobic part and a hydrophilic part of the molecule. The more hydrophobic part of surfactants is frequently derived from (fossil-based or bio-based) fatty acids/-alcohols/-amines, or (fossil based) alkyl-phenols. The hydrophilic part often comprises polar groups like ethylene oxide based oligomers (non-ionic surfactants), or sulfonated /sulphated components (ionic surfactants).

There is a strong desire to replace current fossil-based surfactants, especially anionic surfactants, with fully biobased alternatives which can operate under a wide range of operating conditions (pH, temperature, water hardness, etc). However, most fully biobased surfactants are either hydrolytically unstable (e.g. sorbitan esters), non-ionic in nature (e.g. Alkyl Poly Glycosides), or hard water sensitive (e.g. carboxylate salts of fatty acids / soaps) leading to a limited application potential. Therefore there is a clear need for a wider variety of more hydrolytically stable biobased surfactants, as well as anionic biobased surfactants.

Additionally, particulate soils do not tend to be hydrophobic and as such are less attractive to the typical anionic and nonionic workhorse surfactants. Of particular relevance to this invention are those particulate soils that contain Fe III species as these tend to be highly coloured and are extremely consumer noticeable. Typical red soils containing these particulates can be found throughout the world and so they present a particular and noticeable challenge to the formulator.

Currently, there is an unmet need for specialty surfactants that are biobased, that are biodegradable and that provide distinctive surface active properties.

Accordingly, it is an object of the invention to provide novel (anionic) surface active compounds with distinct and advantageous properties.

It is a particular object of the present invention to provide (anionic) surfactants that are carbohydrate based, preferably based on naturally derived carbohydrates.

It is a further object of the present invention to provide surfactants that are biodegradable

It is a further object of the present invention to provide surfactants that exhibit superior properties over currently existing (biobased) surface active agents, in terms of e.g. foaming, critical micelle concentration, neutral surface tension, alkaline surface tensions, etc.

It is a still further object of the present invention to provide biobased surfactants that particularly stand out in terms of foaming properties.

It is a still further object of the present invention to provide biobased surfactants that particularly stand out in terms of emulsion properties.

It is a still further object of the present invention to provide biobased specialty surfactants, particularly biobased specialty surfactants that stand out in terms of soil removal properties, more particularly red soil removal properties.

### SUMMARY OF THE INVENTION

The inventors have unexpectedly established that one or more of these objectives can be met by employing one or more aldaric acid derivatives as defined herein.

As will be shown in the appended examples, it was surprisingly found that the incorporation of one or more aldaric acid derivatives according to the invention in typical laundry compositions enhance the performance thereof, in particular the ability to remove particulate-soil.

Accordingly, in a first aspect, the invention relates to aldaric acid derivatives having a structure represented by formula (I): 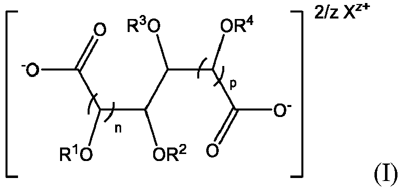 wherein:
*n* is selected from 0 and 1;
*p* is selected from 0 and 1;
   when *n* is 1 and *p* is 1,
   (a) R² is connected to R¹, -R¹ and -R² together form group -C(R⁵R⁶)-, R³ is connected to R⁴, and -R³ and -R⁴ together form group -C(R⁷R⁸)-; or
   (b) R² is connected to R⁴, -R² and -R⁴ together form group -C(R⁵R⁶)-, R¹ is connected to R³, and -R¹ and -R³ together form group -C(R⁷R⁸)-; or
   (c) R² is connected to R³, -R² and -R³ together form group -C(R⁵R⁶)-, R¹ is connected to R⁴, and -R¹ and -R⁴ together form group -C(R⁷R⁸)-; or
   (d) R² is connected to R⁴, -R² and -R⁴ together form group -C(R⁵R⁶)-, and R¹ and R³ are both hydrogen; or
   (e) R² is connected to R¹, -R¹ and -R² together form group -C(R⁵R⁶)-, and R³ and R⁴ are both hydrogen; or
   (f) R² is connected to R³, -R² and -R³ together form group -C(R⁵R⁶)-, and R¹ and R⁴ are both hydrogen; or
   (g) R¹ is connected to R⁴, -R¹ and -R⁴ together form group -C(R⁵R⁶)-, and R² and R³ are both hydrogen; or
      when *n* is 0 and *p* is 1,
   (h) R² is connected to R³, -R² and -R³ together form group -C(R⁵R⁶)-, and R⁴ is hydrogen; or
   (i) R² is connected to R⁴, -R² and -R⁴ together form group -C(R⁵R⁶)-, and R³ is hydrogen; or
      when *n* is 0 and *p* is 0,
   (j) R² is connected to R³, -R² and -R³ together form group -C(R⁵R⁶)-,
wherein R⁵ is selected from the group consisting of saturated or unsaturated C₁-C₁₇ alkyl and wherein R⁶, R⁷ and R⁸ are independently selected from the group consisting of hydrogen and saturated or unsaturated C₁-C₁₇ alkyl, with the proviso that R¹, R², R³ and R⁴ together have 3 to 28 carbon atoms, and
wherein X^{*z*+} is selected from the group consisting of H⁺, metal cation M^{*z*+} with *z* = 1, 2 or 3, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein each R⁹ is individually chosen from the group consisting of aryl, alkyl and alkyl alcohol.

In a second aspect, the invention provides compositions comprising one or more aldaric acid derivatives according to the invention, wherein the one or more aldaric acid derivatives comprise more than 50 wt.% of the composition based on the total weight of the composition.

In a third aspect, a method is provided for preparing aldaric acid derivatives according to formula (I), comprising the steps of:
(i) protecting the carboxylic acid groups of an aldaric acid chosen from the group consisting of C₄, C₅ and C₆ aldaric acids by esterification with an hydroxy-functionalized protecting group, preferably in the presence of an acid catalyst, preferably in a solvent;
(ii) reacting the product obtained in step (i) with an acetal, ketal, aldehyde, ketone or a mixture thereof, preferably in the presence of an acid catalyst, preferably in a solvent;
(iii) deprotecting the carboxylic acid groups of the product obtained in step (ii) via hydrolysis of the esters, preferably by saponification under alkaline conditions;
(iv) optionally further conversion of the deprotected carboxylic acid groups by salt formation or salt conversion.

### DETAILED DESCRIPTION

### Compounds

In a first aspect, the invention concerns aldaric acid derivatives having a structure represented by formula (I): 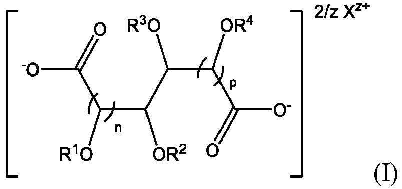 wherein:
*n* is selected from 0 and 1;
*p* is selected from 0 and 1;
   when *n* is 1 and *p* is 1,
   (a) R² is connected to R¹, -R¹ and -R² together form group -C(R⁵R⁶)-, R³ is connected to R⁴, and -R³ and -R⁴ together form group -C(R⁷R⁸)-, resulting in a compound according to formula (I-a); or 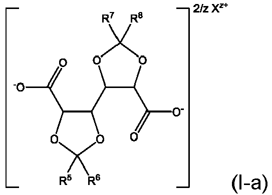
   (b) R² is connected to R⁴, -R² and -R⁴ together form group -C(R⁵R⁶)-, R¹ is connected to R³, and -R¹ and -R³ together form group -C(R⁷R⁸)-, resulting in a compound according to formula (1-b); or 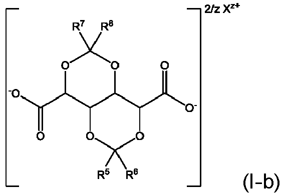
   (c) R² is connected to R³, -R² and -R³ together form group -C(R⁵R⁶)-, R¹ is connected to R⁴, and -R¹ and -R⁴ together form group -C(R⁷R⁸)-, resulting in a compound according to formula (I-c); or 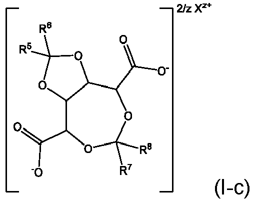
   (d) R² is connected to R⁴, -R² and -R⁴ together form group -C(R⁵R⁶)-, and R¹ and R³ are both hydrogen, resulting in a compound according to formula (I-d); or 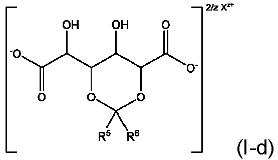
   (e) R² is connected to R¹, -R¹ and -R² together form group -C(R⁵R⁶)-, and R³ and R⁴ are both hydrogen, resulting in a compound according to formula (I-e); or 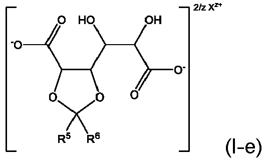
   (f) R² is connected to R³, -R² and -R³ together form group -C(R⁵R⁶)-, and R¹ and R⁴ are both hydrogen, resulting in a compound according to formula (I-f); or 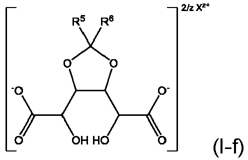
   (g) R¹ is connected to R⁴, -R¹ and -R⁴ together form group -C(R⁵R⁶)-, and R² and R³ are both hydrogen, resulting in a compound according to formula (I-g); or 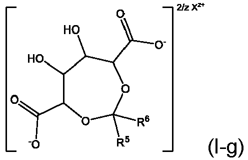 when *n* is 0 and *p* is 1,
   (h) R² is connected to R³, -R² and -R³ together form group -C(R⁵R⁶)-, and R⁴ is hydrogen, resulting in a compound according to formula (I-h); or 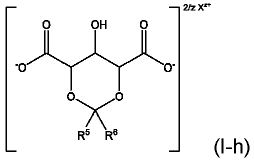
   (i) R² is connected to R⁴, -R² and -R⁴ together form group -C(R⁵R⁶)-, and R³ is hydrogen, resulting in a compound according to formula (I-i); or 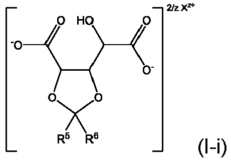 when *n* is 0 *and p* is 0,
   (j) R² is connected to R³, -R² and -R³ together form group -C(R⁵R⁶)-, resulting in a compound according to formula (I-j), 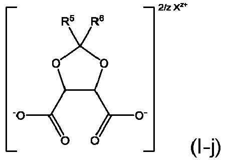
wherein R⁵ is selected from the group consisting of saturated or unsaturated C₁-C₁₇ alkyl and wherein R⁶, R⁷ and R⁸ are independently selected from the group consisting of hydrogen and saturated or unsaturated C₁-C₁₇ alkyl, with the proviso that R¹, R², R³ and R⁴ together have 3 to 28 carbon atoms, and
wherein X^{*z*+} is selected from the group consisting of H⁺, metal cation M^{*z*+} with *z* = 1, 2 or 3, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein each R⁹ is individually chosen from the group consisting of aryl, alkyl and alkyl alcohol.

It will be understood by those skilled in the art, based on the present teachings, that when two of the substituents selected from the group consisting of R¹, R², R³ and R⁴ are connected and those substituents form group -C(R⁵R⁶)- or group -C(R⁷R⁸)-, this leads to the formation of a 5-, 6-, or 7-membered heterocyclic group, more specifically a 5-, 6-, or 7-membered heterocyclic acetal, such as a 1,3-dioxolane, 1,3-dioxane or 1,3-dioxepane.

The term "alkyl" as used herein refers to a linear or branched-chain hydrocarbyl substituent, which, as indicated in the definitions of the various embodiments, may be saturated or unsaturated. Most preferably, in accordance with the invention, these hydrocarbyl substituents are saturated.

In preferred embodiments, the aldaric acid derivative is a compound according to formula (I), wherein R¹, R², R³ and R⁴ together have 6 to 24 carbon atoms, preferably 7 to 18 carbon atoms, even more preferably 8 to 16 carbon atoms.

In preferred embodiments, the compound of the invention is a compound according to formula (I), such as a compound according to any one of formulae (I-a)-(I-j), wherein the alkyl in groups R⁵, R⁶, R⁷ and R⁸ is a linear alkyl.

In preferred embodiments, the aldaric acid derivative is a compound according to formula (I), such as a compound according to any one of formulae (I-a)-(I-j), wherein z is 1 and wherein X⁺ is selected from the group consisting of H⁺, metal cations M⁺, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein X⁺ is preferably selected from H⁺, Na⁺, K⁺, Li⁺, NH₄⁺ and ethanolammonium, wherein X⁺ more preferably is Na⁺.

Preferred aldaric acid derivatives according to the present invention have a structure represented by formulas (II-a), (II-b), (II-c), (II-d), (II-e), (II-f) or (II-g): 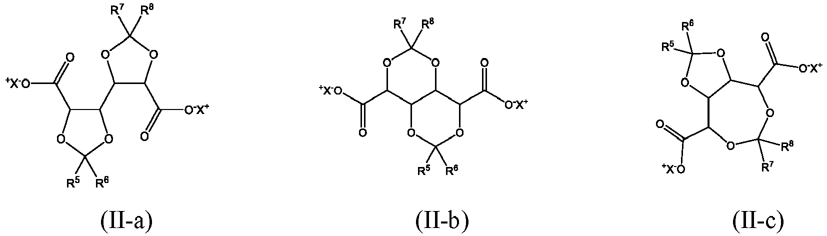 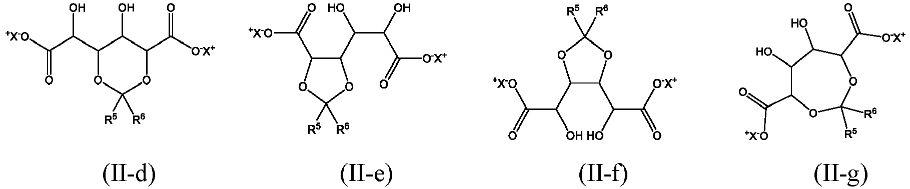 wherein X⁺ is selected from the group consisting of H⁺, metal cations M⁺, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein X⁺ is preferably selected from H⁺, Na⁺, K⁺, Li⁺, NH₄⁺ and ethanolammonium, wherein X⁺ more preferably is Na⁺.

In preferred embodiments, the aldaric acid derivative has a structure represented by formula (II-a), (II-b) or (II-c), wherein R⁶ and R⁸ are hydrogen and wherein R⁵ and R⁷ are independently selected from the group consisting of saturated or unsaturated C₃-C₂₀ alkyl, preferably saturated or unsaturated C₃-C₁₂ alkyl, more preferably saturated or unsaturated C₄-C₉ alkyl.

In preferred embodiments, the aldaric acid derivative has a structure represented by formula (II-a), (II-b) or (II-c) wherein R⁵ and R⁷ are identical.

In preferred embodiments, the aldaric acid derivative has a structure represented by formula (II-a), (II-b) or (II-c), wherein R⁶ and R⁸ are hydrogen and wherein R⁵ and R⁷ are identical and selected from the group consisting of saturated or unsaturated C₃-C₂₀ alkyl, preferably saturated or unsaturated C₃-C₁₂ alkyl, more preferably saturated or unsaturated C₄-C₉ alkyl.

In preferred embodiments, the aldaric acid derivative has a structure represented by formula (II-a), (II-b) or (II-c), wherein R⁵, R⁶, R⁷ and R⁸ are independently selected from the group consisting of saturated or unsaturated C₁-C₂₀ alkyl, preferably saturated or unsaturated C₁-C₁₂ alkyl, more preferably saturated or unsaturated C₁-C₉ alkyl.

In preferred embodiments, the aldaric acid derivative has a structure represented by formula (II-a), (II-b) or (II-c), wherein R⁵ and R⁷ are identical and wherein R⁶ and R⁸ are identical and wherein R⁵ and R⁶ are independently selected from the group consisting of saturated or unsaturated C₁-C₂₀ alkyl, preferably saturated or unsaturated C₁-C₁₂ alkyl, more preferably saturated or unsaturated C₁-C₉ alkyl.

In preferred embodiments, the aldaric acid derivative has a structure represented by formula (II-d), (II-e), (II-f) or (II-g), wherein R⁶ is hydrogen and wherein R⁵ is selected from the group consisting of saturated or unsaturated C₃-C₂₀ alkyl, preferably saturated or unsaturated C₃-C₁₆ alkyl, more preferably saturated or unsaturated C₄-C₁₂ alkyl.

In preferred embodiments, the aldaric acid derivative has a structure represented by formula (II-d), (II-e), (11-f) or (II-g), wherein R⁵ and R⁶ are independently selected from the group consisting of saturated or unsaturated C₁-C₂₀ alkyl, preferably saturated or unsaturated C₁-C₁₂ alkyl, more preferably saturated or unsaturated C₁-C₉ alkyl.

In preferred embodiments, the aldaric acid derivative has a structure represented by formula (II-d), (II-e), (II-f) or (II-g), wherein R⁶ is hydrogen and wherein R⁵ is selected from the group consisting of C₃-C₁₂ alkyl, preferably R⁵ is selected from the group consisting of prop-1-yl, but-1-yl, pent-1-yl, hex-1-yl, hept-1-yl, hept-3-yl, oct-1-yl, non-1-yl, more preferably from the group consisting of but-1-yl, hept-1-yl, oct-1-yl and non-1-yl.

In preferred embodiments, the aldaric acid derivative has a structure represented by formula (II-a), (II-b) or (II-c), wherein R⁶ and R⁸ are hydrogen and wherein R⁵ and R⁷ are independently selected from the group consisting of C₃-C₉ alkyl, preferably R⁵ and R⁷ are independently selected from the group consisting of prop-1-yl, but-1-yl, pent-1-yl, hex-1-yl, hept-1-yl, hept-3-yl, oct-1-yl, non-1-yl, more preferably from the group consisting of but-1-yl, hept-1-yl, oct-1-yl and non-1-yl.

As will be evident to those skilled in the art, based on the present teachings, the aldaric acid derivatives of the formula (I) comprise numerous centers of chirality. The invention is not particularly limited with regard to the orientation of these chiral centers. Nonetheless, in accordance with the invention, it is particularly preferred to produce the aldaric acid derivatives of the formula (I) from biomass sources, and there is a particular configuration that is inherent to such compounds as obtained from biomass sources. In preferred embodiments, the aldaric acid derivatives of the formula (I) have a configuration that corresponds to the configuration of a C₄, C₅ or C₆ aldaric acid, such as arabinaric acid, lyxaric acid, ribaric acid, xylaric acid, allaric acid, altraric acid, galactaric acid, glucaric acid, gularic acid, idaric acid, mannaric acid, talaric acid and tartaric acid.

In preferred embodiments, the aldaric acid derivatives of the formula (II-a), (II-b), (II-c), (II-d), (II-e), (II-f) or (II-g) have the galacto configuration or have the following stereochemistry:

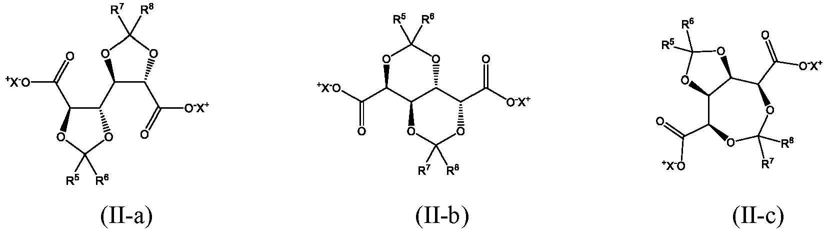

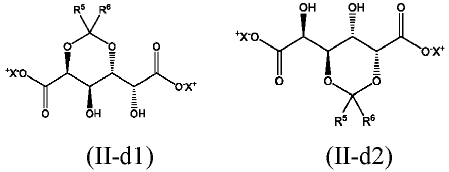

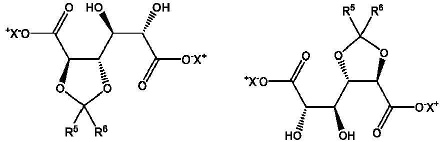

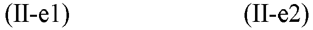

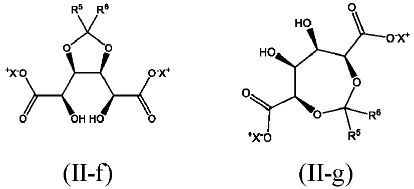

### Compositions

In a second aspect, the invention concerns compositions comprising one or more aldaric derivatives of the invention, wherein the one or more aldaric acid derivatives make up more acid than 50 wt.% of the composition based on the total weight of the composition, preferably more than 60 wt.% of the composition based on the total weight of the composition, such as more than 75 wt.%, more than 85 wt.%, more than 90 wt.%, more than 92.5 wt.%, more than 95 wt.%, more than 96 wt.%, more than 97 wt.%, more than 98 wt.% or more than 99 wt.%. Compositions comprising the compounds of the invention as the major component are suitable as an ingredient in, e.g., the production of laundry compositions, particularly laundry composition with improved soil removal from fabrics. It will be apparent to the skilled person, based on the present teachings, how to suitably formulate the aldaric acid derivatives of the invention, depending on the exact end-application(s) that is/are envisaged.

In preferred embodiments, compositions are provided comprising one or more aldaric acid derivatives of formula (I), such as one or more aldaric acid derivatives according to any one of formulae (I-a)-(I-j), wherein R⁵ is identical in all aldaric acid derivatives of formula (I) present in the composition, R⁶ is identical in all aldaric acid derivatives of formula (I) present in the composition, R⁷ is identical in all aldaric acid derivatives of formula (I) present in the composition and R⁸ is identical in all aldaric acid derivatives of formula (I) present in the composition.

In preferred embodiments, compositions are provided comprising one or more aldaric acid derivatives of formula (I), such as one or more aldaric acid derivatives according to any one of formulae (I-a)-(I-j), wherein R⁵ and R⁷ are hydrogen in all aldaric acid derivatives of formula (I) present in the composition, R⁶ is identical in all aldaric acid derivatives of formula (I) present in the composition and R⁸ is identical in all aldaric acid derivatives of formula (I) present in the composition.

In preferred embodiments, compositions are provided comprising one or more aldaric acid derivatives of formula (I), such as one or more aldaric acid derivatives according to any one of formulae (I-a)-(I-j), wherein R⁵ and R⁷ are hydrogen in all aldaric acid derivatives of formula (I) present in the composition, and R⁶ and R⁸ are identical to each other and identical in all aldaric acid derivatives of formula (I) present in the composition.

In preferred embodiments, compositions are provided, wherein the one or more aldaric acid derivatives of the invention are selected from:
- one aldaric acid derivative according to formula (I-a);
- one aldaric acid derivative according to formula (I-b);
- one aldaric acid derivative according to formula (I-c);
- one aldaric acid derivative according to formula (I-d);
- one aldaric acid derivative according to formula (I-e);
- one aldaric acid derivative according to formula (I-f);
- one aldaric acid derivative according to formula (I-g);
- one aldaric acid derivative according to formula (I-h);
- one aldaric acid derivative according to formula (I-i); and
- one aldaric acid derivative according to formula (I-j);
wherein, in all said formulas (I-a)-(I-j), R⁵ has the same meaning, R⁶ has the same meaning, R⁷ has the same meaning and R⁸ has the same meaning. Preferably, in all said formulas (I-a)-(I-j), R⁶ has the same meaning, R⁸ has the same meaning, R⁵ is hydrogen, and R⁷ is hydrogen. More preferably in all said formulas (I-a)-(I-j), R⁶ has the same meaning, R⁸ has the same meaning, R⁵ represents hydrogen and R⁷ represent hydrogen, and, in each formula, R⁶ and R⁸ have the same meaning. As will be understood by those skilled in the art, based on the present teachings, the compositions according to this particular embodiment can typically be produced from an C₄, C₅, C₆ aldaric acid or a mixture thereof and an acetal, ketal, aldehyde or ketone or a mixture thereof, employing the methods of the present invention.

In particularly preferred embodiments, compositions as defined here above are provided, wherein the one or more aldaric acid derivatives of the invention are selected from:
- one aldaric acid derivative according to formula (I-a);
- one aldaric acid derivative according to formula (I-b);
- one aldaric acid derivative according to formula (I-c);
- one aldaric acid derivative according to formula (I-d);
- one aldaric acid derivative according to formula (I-e);
- one aldaric acid derivative according to formula (1-f); and
- one aldaric acid derivative according to formula (I-g);
wherein, in all said formulas (I-a)-(I-g), R⁵ has the same meaning, R⁶ has the same meaning, R⁷ has the same meaning, and R⁸ has the same meaning. Preferably, in all said formulas (I-a)-(I-g), R⁶ has the same meaning, R⁸ has the same meaning, R⁵ is hydrogen, and R⁷ is hydrogen. More preferably in all said formulas (I-a)-(I-j), R⁶ has the same meaning, R⁸ has the same meaning, R⁵ represents hydrogen and R⁷ represent hydrogen, and, in each formula, R⁶ and R⁸ have the same meaning. As will be understood by those skilled in the art, based on the present teachings, the compositions according to this particular embodiment can typically be produced from a C₆ aldaric acid and an acetal, ketal, aldehyde or ketone or a mixture thereof, employing the methods of the present invention.

More preferably, compositions as defined here above are provided, wherein two or more of the recited aldaric acid derivatives (I-a) - (I-g) are present, more preferably three or more, four or more, five or more, or six or more of the recited aldaric acid derivatives (I-a) - (I-g) are present. In a further preferred embodiment of the invention, two, three, four, five, six or all seven of the recited aldaric acid derivatives (I-a) - (I-g) are present.

In preferred embodiments, compositions as defined here above are provided, wherein the one aldaric acid derivative according to formula (I-a), the one aldaric acid derivative according to formula (I-b) and the one aldaric acid derivative according to formula (I-c) together make up at least 85 wt.% of the total amount of aldaric acid derivatives of the invention that are present in the composition, preferably at least 90 wt.%, such as at least 91 wt.%, at least 92 wt.%, at least 93 wt.%, at least 94 wt.%, at least 95 wt.%, at least 96 wt.%, at least 97 wt.%, at least 98 wt.%, or at least 99 wt.% of the total amount of aldaric acid derivatives of the invention that are present in the composition.

In preferred embodiments, compositions as defined here above are provided, wherein the one aldaric acid derivative according to formula (I-d), the one aldaric acid derivative according to formula (I-e), the one aldaric acid derivative according to formula (I-f) and the one aldaric acid derivative according to formula (I-g) together make up at least 85 wt.% of the total amount of aldaric acid derivatives of the invention that are present in the composition, preferably at least 90 wt.%, such as at least 91 wt.%, at least 92 wt.%, at least 93 wt.%, at least 94 wt.%, at least 95 wt.%, at least 96 wt.%, at least 97 wt.%, at least 98 wt.%, or at least 99 wt.% of the total amount of aldaric acid derivatives of the invention that are present in the composition.

In preferred embodiments, compositions as defined here above are provided, wherein the ratio between the combined weight of the one aldaric acid derivative according to formula (I-a), the one aldaric acid derivative according to formula (I-b) and the one aldaric acid derivative according to formula (I-c) and the combined weight of the one aldaric acid derivative according to formula (I-d), the one aldaric acid derivative according to formula (I-e), the one aldaric acid derivative according to formula (I-f) and the one aldaric acid derivative according to formula (I-g) is higher than 1:1, preferably higher than 2: 1, more preferably higher than 3: 1, even more preferably higher than 4: 1, such as higher than 5:1, higher than 6: 1, higher than 7: 1, higher than 8:1, higher than 9:1, higher than 10:1, higher than 15:1, higher than 20:1, higher than 25:1, higher than 50:1, higher than 75:1, higher than 100:1.

In other preferred embodiments, compositions as defined here above are provided, wherein the ratio between the combined weight of the one aldaric acid derivative according to formula (I-d), the one aldaric acid derivative according to formula (I-e), the one aldaric acid derivative according to formula (I-f) and the one aldaric acid derivative according to formula (I-g) and the combined weight of the one aldaric acid derivative according to formula (I-a), the one aldaric acid derivative according to formula (I-b) and the one aldaric acid derivative according to formula (I-c) is higher than 1: 1, preferably higher than 2: 1, more preferably higher than 3:1, even more preferably higher than 4: 1, such as higher than 5:1, higher than 6: 1, higher than 7:1, higher than 8:1, higher than 9:1, higher than 10:1, higher than 15:1, higher than 20:1, higher than 25: 1, higher than 50: 1, higher than 75:1, higher than 100:1.

In further particularly preferred embodiments of the invention, compositions as defined herein are provided, wherein the one or more aldaric acid derivatives of the invention are selected from:
- one aldaric acid derivative according to formula (I-a);
- one aldaric acid derivative according to formula (I-b); and
- one aldaric acid derivative according to formula (I-c).
wherein, in all said formulas (I-a), (I-b) and (I-c), R⁵ has the same meaning, R⁶ has the same meaning, R⁷ has the same meaning and R⁸ has the same meaning. Preferably, in all said formulas (I-a), (I-b) and (I-c), R⁶ has the same meaning, R⁸ has the same meaning, R⁵ is hydrogen, and R⁷ is hydrogen. More preferably in all said formulas (I-a), (I-b), (I-c), R⁶ has the same meaning, R⁸ has the same meaning, R⁵ represents hydrogen and R⁷ represent hydrogen, and, in each formula, R⁶ and R⁸ have the same meaning. As will be understood by those skilled in the art, based on the present teachings, the compositions according to this particular embodiment can typically be produced from a C₆ aldaric acid and an acetal, ketal, aldehyde or ketone or a mixture thereof, employing the methods of the present invention.

More preferably, in the compositions as defined here above, two or more of the recited aldaric acid derivatives (I-a) - (I-c) are present. In a further preferred embodiment of the invention, two or three of the recited aldaric acid derivatives (I-a) - (I-c) are present.

In yet further preferred embodiments, compositions as defined herein are provided, wherein the one or more aldaric acid derivatives of the invention are selected from:
- one aldaric acid derivative according to formula (I-d);
- one aldaric acid derivative according to formula (I-e);
- one aldaric acid derivative according to formula (I-f);
- one aldaric acid derivative according to formula (I-g);
wherein, in all said formulas (I-d)-(I-g), R⁵ has the same meaning and R⁶ has the same meaning. Preferably, in all said formulas (I-d)-(I-g), R⁶ has the same meaning and R⁵ is hydrogen. As will be understood by those skilled in the art, based on the present teachings, the compositions according to this particular embodiment can typically be produced from a C₆ aldaric acid and an acetal, ketal, aldehyde or ketone or a mixture thereof, employing the methods of the present invention.

More preferably, in the compositions as defined here above, two or more of the recited aldaric acid derivatives (I-d) - (I-g) of the invention are present, more preferably three or more of the recited aldaric acid derivatives (I-d) - (I-g) are present. In a further preferred embodiment of the invention, two, three or all four of the recited aldaric acid derivatives (I-d) - (I-g) are present.

In preferred embodiments of the invention, compositions as defined herein are provided, wherein the one aldaric acid derivative according to formula (I-a) is a aldaric acid derivative according to formula (II-a).

In preferred embodiments of the invention, compositions as defined herein are provided, wherein the one aldaric acid derivative according to formula (I-b) is a aldaric acid derivative according to formula (II-b)

In preferred embodiments of the invention, compositions as defined herein are provided, wherein the one aldaric acid derivative according to formula (I-c) is a aldaric acid derivative according to formula (II-c)

In preferred embodiments of the invention, compositions as defined herein are provided, wherein the one aldaric acid derivative according to formula (I-d) is a aldaric acid derivative according to formula (II-d)

In preferred embodiments of the invention, compositions as defined herein are provided, wherein the one aldaric acid derivative according to formula (I-e) is a aldaric acid derivative according to formula (II-e)

In preferred embodiments of the invention, compositions as defined herein are provided, wherein the one aldaric acid derivative according to formula (I-f) is a aldaric acid derivative according to formula (II-f)

In preferred embodiments of the invention, compositions as defined herein are provided, wherein the one aldaric acid derivative according to formula (I-g) is a aldaric acid derivative according to formula (II-g)

In preferred embodiments, the one or more aldaric acid derivatives of the invention are selected from:
- one aldaric acid derivative according to formula (I-h); and
- one aldaric acid derivative according to formula (I-i);
wherein, in formulas (I-h) and (I-i), R⁵ has the same meaning and R⁶ has the same meaning. Preferably, in formulas (I-h) and (I-i), R⁵ is hydrogen and R⁶ has the same meaning. As will be understood by those skilled in the art, based on the present teachings, the compositions according to this particular embodiment can typically be produced from a C₅ aldaric acid and an acetal, ketal, aldehyde or ketone or a mixture thereof, employing the methods of the present invention.

In embodiments, the composition as defined herein has a water content below 5 wt.% based on the total weight of the composition, such as a water content below 4 wt.%, below 3 wt.%, below 2 wt.%, below 1 wt.%.

### Methods of preparation

In a third aspect, the invention concerns a method for preparing the aldaric acid derivative or compositions of the invention, said method comprising the steps of:
(i) providing an aldaric acid chosen from the group consisting of C₄, C₅ and C₆ aldaric acids;
(ii) protecting the carboxylic acid groups of the aldaric acid by esterification with an hydroxy-functionalized protecting group, preferably in the presence of an acid catalyst, preferably in a solvent;
(iii) reacting the product obtained in step (i) with an acetal, ketal, aldehyde, ketone or a mixture thereof, preferably in the presence of an acid catalyst, preferably in a solvent;
(iv) deprotecting the carboxylic acid groups of the product obtained in step (ii) via hydrolysis of the esters, preferably by saponification under alkaline conditions;
(v) optionally further conversion of the deprotected carboxylic acid groups by salt formation or salt conversion.

In accordance with the invention, it is preferred that the C₄, C₅ and C₆ aldaric acid provided in step (i) is derived from a biomass source.

It is known by those skilled in the art, how to obtain the C₄, C₅ and C₆ aldaric acid provided in step (i) through the oxidation of the corresponding aldose, uronic acid or aldaric acid. A highly efficient process for the oxidative conversion of galacturonic acid to galactaric acid, has recently been disclosed in international patent applications WO 2013/151428 and WO 2016/056907, the contents of which are incorporated herein by reference.

In preferred embodiments of the invention, methods as defined herein are provided, wherein the C₄, C₅ or C₆ aldaric acid provided in step (i) is chosen from the group consisting of arabinaric acid, lyxaric acid, ribaric acid, xylaric acid, allaric acid, altraric acid, galactaric acid, glucaric acid, gularic acid, idaric acid, mannaric acid, talaric acid and tartaric acid.

In preferred embodiments of the invention, methods as defined herein are provided, wherein the hydroxy-functionalized protecting group of step (ii) is chosen from the group consisting of C₁-C₄-alkyl alcohol, preferably chosen from the group consisting of methanol, ethanol, isopropyl alcohol, *n*-butyl alcohol, isobutyl alcohol and *tert*-butyl alcohol.

In preferred embodiments of the invention, methods as defined herein are provided, wherein the hydroxy-functionalized protecting group of step (ii) and the solvent used in step (ii) are identical and are selected from the group consisting of C₁-C₄-alkyl alcohol, preferably chosen from the group consisting of methanol, ethanol, isopropyl alcohol, *n*-butyl alcohol, isobutyl alcohol and *tert-*butyl alcohol.

In preferred embodiments of the invention, methods as defined herein are provided, wherein the acid catalyst used in step (ii) is HCl or H₂SO₄

In preferred embodiments of the invention, methods as defined herein are provided, wherein step (ii) is performed at a temperature between 0°C and 200°C, preferably at a temperature between 20°C and 150°C.

In preferred embodiments of the invention, methods as defined herein are provided, wherein the reaction time for step (ii) is between 1 and 48 hours, preferably between 12 and 36 hours

In preferred embodiments of the invention, methods as defined herein are provided, wherein the amount of hydroxy-functionalized protecting group used in step (ii) is controlled such that the molar ratio of the hydroxy-functionalized protecting group to the aldaric acid is within the range of 10 to 150, preferably within the range of 50 to 100.

In preferred embodiments of the invention, step (iii) comprises reacting the product obtained in step (i) with an aldehyde or ketone represented by formula (III-a) and/or (III-b): 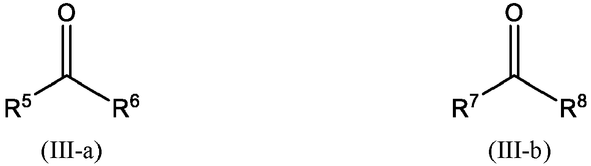 wherein R⁵, R⁶, R⁷ and R⁸ are as defined herein (in relation to formulas (I) and/or (II)) or with an acetal or ketal derived from said aldehyde or ketone represented by formula (III-a) and/or (III-b), or with any mixture of such aldehydes, ketones, acetals and/or ketals, preferably in the presence of an acid catalyst, preferably in a solvent.

In preferred embodiments of the invention, methods as defined herein are provided, wherein the acetal, ketal, aldehyde, ketone used in step (iii) is selected from the group consisting of branched or linear, saturated or unsaturated C₁-C₁₇ aldehyde or ketone, and the corresponding dialkoxylated, preferably dimethoxylated or diethoxylated, acetals or ketals thereof, preferably selected from the group consisting of branched or linear butanone, butanal, pentanone, pentanal, hexanone, hexanal, heptanone, heptanal, octanone, octanal, nonanone, nonanal, decanone, decanal and the corresponding dialkoxylated, preferably dimethoxylated or diethoxylated, acetals or ketals thereof, more preferably selected from the group consisting of linear butanal, heptanal, octanal, decanal and the corresponding dialkoxylated, preferably dimethoxylated or diethoxylated, acetals or ketals thereof.

In preferred embodiments of the invention, methods as defined herein are provided, wherein the amount of acetal, ketal, aldehyde, ketone or a mixture thereof used in step (iii) is controlled such that the molar ratio of the acetal, ketal, aldehyde, ketone or a mixture thereof to the product obtained after step (ii) is within the range of 0.3 to 10, preferably within the range of 0.5 to 5.

In preferred embodiments of the invention, methods as defined herein are provided, wherein the acid catalyst used in step (iii) is H₂SO₄, methanesulfonic acid, *p*-toluenesulfonic acid.

In preferred embodiments of the invention, methods as defined herein are provided, wherein after step (iii) the acid catalyst used in step (iii) is neutralized.

In preferred embodiments of the invention, methods as defined herein are provided, wherein the solvent used in step (iii) is a non polar solvent (e.g. toluene), an aprotic green organic solvent, a DES (deep eutectic solvent) or an ionic liquid.

In preferred embodiments, the acetal, ketal, aldehyde, ketone used in step (iii) and the solvent used in step (iii) are identical.

In preferred embodiments of the invention, methods as defined herein are provided, wherein step (iii) is performed at a temperature between 0°C and 200°C, preferably at a temperature between 20°C and 150°C.

In preferred embodiments of the invention, methods as defined herein are provided, wherein the reaction time for step (iii) is between 0.1 and 36 hours, preferably between 0.5 and 24 hours.

In preferred embodiments of the invention, methods as defined herein are provided, wherein the alkaline conditions used in step (iv) are realized using a sodium hydroxide of potassium hydroxide.

In preferred embodiments of the invention, methods as defined herein are provided, wherein step (iv) is performed at a temperature between 0°C and 200°C, preferably at a temperature between 20°C and 150°C.

In preferred embodiments of the invention, methods as defined herein are provided, wherein the reaction time for step (iv) is between 0.1 and 10 hours, preferably between 0.5 and 5 hours.

In preferred embodiments of the invention, methods as defined herein are provided, wherein the method comprises a purification step, whereby this purification step is performed:
(A) between step (ii) and step (iii); or
(B) between step (iii) and step (iv); or
(C) after step (iv); or
(D) a combination of two or more of (A)-(C).

In preferred embodiments of the invention, methods as defined herein are provided, wherein the method comprises one or more separation steps, whereby these separation steps are performed between step (iii) and step (iv) and/or after step (iv). These separation steps are used to separate and/or isolate of one or more aldaric acid derivatives according to the invention from the reaction mixture, by any suitable technique known by the person skilled in the art, such as chromatographic separation, distillation, membrane filtration and/or crystallization.

Based on the present teachings, it is within the normal, routine capabilities of those skilled in the art to determine the appropriate conditions for carrying out the process and to optimize it in terms of yield, selectivity, efficiency, etc. It is well known to those skilled in the art how reaction conditions, including reaction time, reagent ratio, temperature, solvent, separation steps affect these aforementioned parameters.

### EXAMPLES

### Analytical Methods

**GC-MS** analyses was performed on a Interscience Trace 1300 with AS3000 II auto sampler (He-carrier gas, flow 1.2 mL/min, split flow 50 mL/min; Restek GC column Rxi-5ms 30 m x 0.25 mm x 0.25 µm; GC program: hold 2 min at 70 °C, ramp 10.0 °C/min, final temperature 300 °C) connected to an Interscience Trace ISQ 7000 (EI, mass range 35-800 Dalton, 200 Ms sample speed). Sample preparation: before analysis the sample was silylated by dissolving the sample into pyridine, adding N,O-Bis(trimethylsilyl)trifluoroacetamide (BSTFA) and subsequently heating the sample to 70 °C for 15 min.

**¹H-NMR** (400.17 MHz) & 13C NMR (100.62 MHz) were recorded on a Bruker Avance III spectrometer and internally referenced to the residual solvent signal. NMR spectra were recorded at 298K.

### Example 1: synthesis of diethyl galactarate

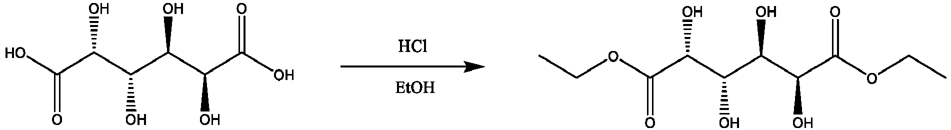

Before use the reactor was first cleaned with boiling water followed by boiling ethanol to remove most traces of water. Then, before the reactor was cooled down, ethanol(7892.00 g, 171.291 mol), galactaric acid(420.00 g, 1.939 mol, 97%) and hydrogen chloride (11.6M) (84.587 g, 2.32 mol) were added. The temperature of the resulting suspension was around 40°C. The reaction mixture was heated to reflux, which was reached 50 min after the reagents were added into the reaction. After 22 hours the temperature of the heating mantle was lowered to 80°C. When the reflux ceased the reaction mixture was drained from the bottom into a Buchner filter on top of a suction flask. The residue from this filtration was dried in vacuo at 40 °C until a constant weight. Analyses showed that the obtained powder was pure galactaric acid (80.300 g, 0.382 mol).

The filtrate was drained into two 5 L bottles and set away into a cooling cell (5°C) for 4 days. Both bottles were filtrated separately and washed with 500 mL ethanol. The collected powder was combined and dried in vacuo at 40 °C until a constant weight was obtained: 373.7 grams. Analyses showed that the obtained white powder is pure diethyl galactarate (373.700 g, 1.404 mol) yield: 72.4%.

¹H NMR (400 MHz, DMSO) δ 4.84 (d, J = 8.0 Hz, 2H), 4.79 (dd, J = 6.1, 2.6 Hz, 2H), 4.33 - 4.26 (m, 2H), 4.12 (qt, J = 7.1, 3.7 Hz, 4H), 3.85 - 3.74 (m, 2H), 1.21 (t, J = 7.1 Hz, 6H). 13C NMR (101 MHz, DMSO) δ 174.11, 71.70, 70.64, 60.57, 14.65.

### Example 2: synthesis of GalX-octyl-di-acetals

### Example 2.1: synthesis of diEt-GalX-octyl-di-acetals

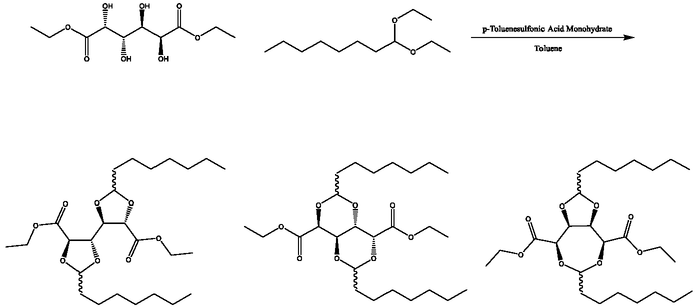

A 250mL round bottom flask was charged with 1,1-diethoxy-octane (cas 54889-48-4, 99%) (21.279 g, 104.114 mmol, 2.800 eq); diethyl galactarate (10.000 g, 37.184 mmol, 1.000 eq); p-Toluenesulfonic acid monohydrate (0.359 g, 1.859 mmol, 0.050 eq) and 100 ml toluene. The resulting suspension was heated to reflux for 22 hours. The reaction mixture was cooled to room temperature, and the toluene solution was washed with 100 ml sodium bicarbonate, and 100 mL brine. The organic layer was dried over MgSO₄ and the solvent was removed in vacuo resulting in 23.63 g of a brown sticky semi-solid.

GC-MS (EI): m/z 485 [M-H]⁺. The GC-MS spectrum revealed 3 closely eluting peaks with an equal molecular ion, corresponding to the 3 different diEt-GalX-octyl-di-acetals, and only a small peak corresponding to the diEt-GalX-octyl-mono-acetals.

### Example 2.2: saponification of diEt-GalX-octyl-di-acetal

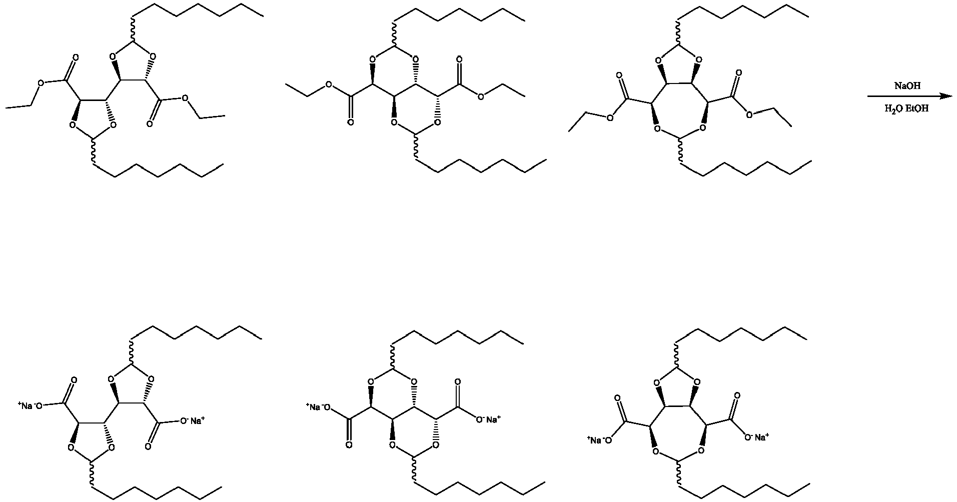

diEt-GalX-octyl-di-acetals (5.000 g, 7.870 mmol, 1.000 eq) was dissolved in a 1:1 mixture of ethanol (12.5 mL) and water (12.5 mL). The mixture was stirred to give an almost clear yellow solution. Next, NaOH (0.800 g, 19.601 mmol, 2.491 eq) was added, and the resulting mixture was heated to reflux (mantle temperature 90 °C). After 2h at reflux, a lighter yellow clear solution formed, together with a dark-red organic layer, and heating was stopped. The solution was cooled to room temperature, and the ethanol was removed using a rotary evaporator. The resulting mixture was added dropwise into a vigorously stirred beaker containing 200 mL acetone. Slight yellow solid particles formed during addition. Stirring was stopped after 30 min, and the solid powder was collected by filtration over a type-3 paper filter, and dried in a vacuum oven, at 40 °C, until constant weight. The final product, diNa-GalX-octyl-di-acetal (4.11 g), was obtained as an off white powder. The outcome of this reaction was confirmed using the ethyl signals in the ¹H-NMR.

### Example 3: synthesis of GalX-octyl-mono-acetal

### Example 3.1: synthesis and separation of diEt-GalX-octyl-mono-acetal

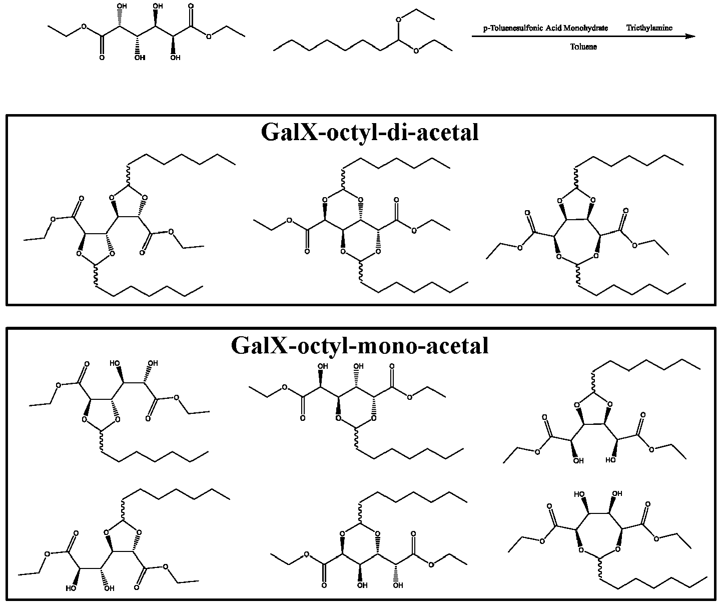

1,1-diethoxy-octane (cas 54889-48-4, 99%) (13.122 g, 0.049 mol, 1.500 eq), diethyl galactarate (8.900 g, 0.032 mol, 1.000 eq,) and p-toluenesulfonic acid monohydrate (0.313 g, 0.002 mol, 0.050 eq) were dissolved in toluene(58.1 mL) in a 250 mL round bottom flask and heated to reflux for 24 hours. The resulting dark red clear solution was cooled to room temperature and triethyl amine (0.225 mL, 0.002 mol, 0.050 eq) was added for neutralization. The toluene was removed on the rotary evaporator, yielding a red oil that solidifies upon cooling.

The resulting solid mass was dissolved in ethyl acetate (100 mL) and washed with water (2 x 100 mL and brine (1 x 50 mL). The red organic layer was dried over MgSO₄ and filtrated. The solvent was removed from the filtrate in vacuo, yielding a red liquid that crystalized into a brown solid mass (16.27g) over night.

The crude solid product was dissolved in a minimal amount of chloroform, and silica gel was added until the sample was dry. The chloroform was removed under reduced pressure at a rotary evaporator, to give the silica-coated product as a dry powder. The silica-coated sample was place in a solid loader.

Purification was performed on a Buchi Reveleris, using hexane / EtOAc as the eluent (0-40% EtOAc). Purification was performed over a 120g silica gel column, using the solid loader. Samples were fractionated according to the ELSD detector, and checked afterwards by TLC (Heptane: EtOAc = 6:4); stained with permanganate.

The resulting diEt-GalX-octyl-di-acetal fractions were combined into a yellow / orange solid (9.85g yield 59%). The resulting diEt-GalX-octyl-mono-acetal fractions were combined into a slight yellow oil (2.92g yield 23%), which crystalized over 2 days at RT.

The GC-MS spectrum of the diEt-GalX-octyl-di-acetal fraction revealed closely eluting peaks with an equal molecular ion, corresponding to the different diEt-GalX-octyl-di-acetals, and no peak corresponding to the diEt-GalX-octyl-mono-acetals. GC-MS (EI): m/z 485 [M-H]+.

The GC-MS spectrum of the diEt-GalX-octyl-mono-acetal fraction revealed closely eluting peaks with an equal molecular ion, corresponding to the different diEt-GalX-octyl-mono-acetals, and no peak corresponding to the diEt-GalX-octyl-di-acetals. GC-MS (EI): m/z 505 [M-CH₃]⁺.

### Example 3.2: saponification of diEt-GalX-octyl-mono-acetal

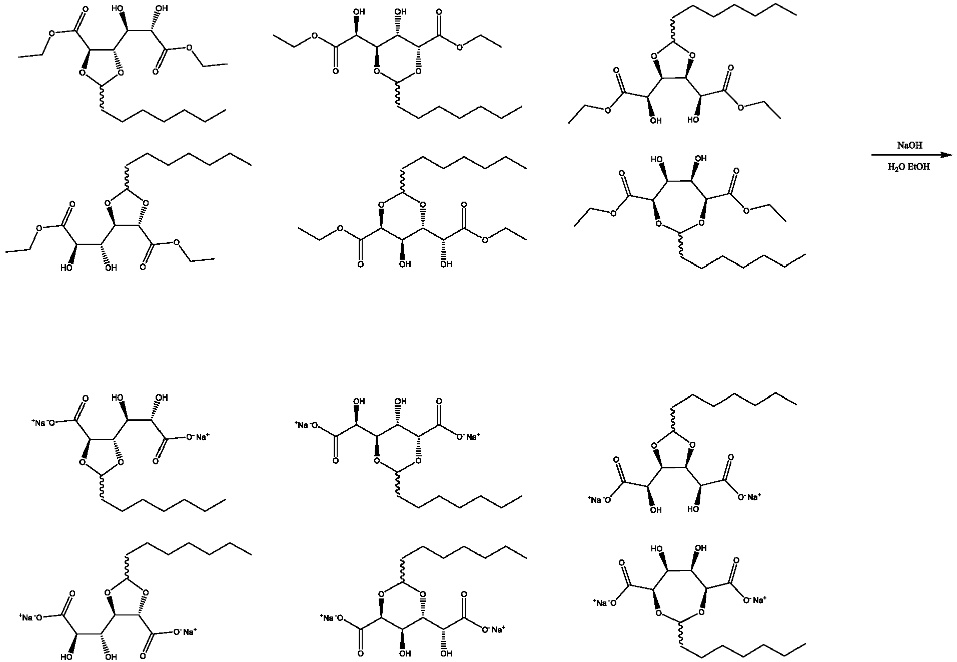

DiEt-GalX-octyl-mono-acetal (2.920 g, 0.008 mol, 1.000 eq) was dissolved in a 1:1 mixture of ethanol (10 mL) and water (10 mL) in a 250 mL round bottom flask. The mixture was stirred to give an almost clear colorless solution. Next, NaOH (0.614 g, 0.015 mol, 2.000 eq) was added, and the resulting mixture was heated to reflux (mantle temperature 90 °C). After 2h, the solution cooled to room temperature, and the ethanol was removed using a rotary evaporator. The resulting mixture was added dropwise into a vigorously stirred beaker containing 100 mL acetone. White solids formed during addition. Stirring was stopped after 30 min, the acetone was decanted, and 100 mL fresh acetone was added. After stirring for 30 min, the resulting white solid powder was collected by filtration over a type-3 glass filter, and dried in a vacuum oven, at 40 °C, until constant weight. The final product, GalX-octyl-mono-acetal (1.94 g yield 71%) was obtained as a white powder.

### Example 4: synthesis of GalX-octyl-mono-acetal

### Example 4.1: synthesis and purification of diEt-GalX-octyl-mono-acetal

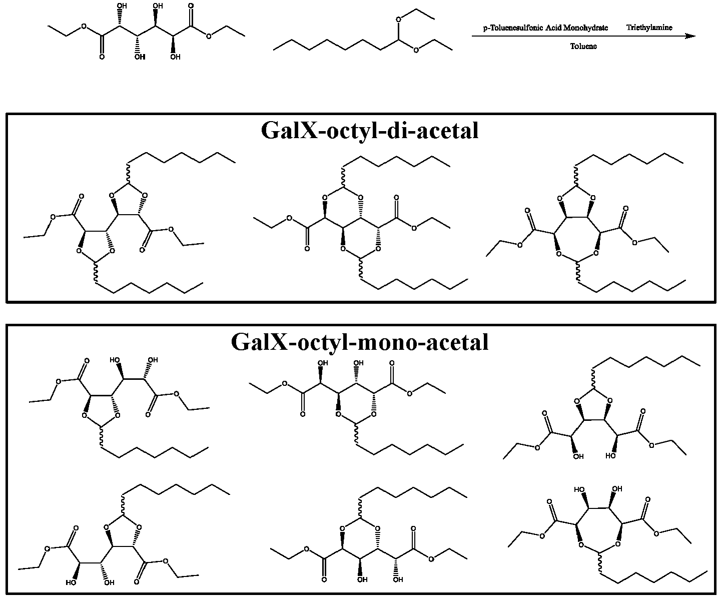

1,1-diethoxy-octane (cas 54889-48-4, 99%) (32.162 g, 0.159 mol, 2.400 eq) and p-toluenesulfonic acid monohydrate (76.7 mg, 0.397 mmol, 0.006 eq) were dissolved in toluene (359.9 mL) in a 1000ml round bottom flask. Subsequently diethyl galactarate (19.815 g, 0.066 mol, 1.000 eq) was added to the reaction solution. The reaction was heated to 130°C and was stirred at reflux for 2h. The reaction mixture was then cooled down to room temperature and triethylamine (0.055 mL, 0.397 mmol, 0.006 eq) was added to neutralize the p-toluenesulfonic acid. The clear yellow solution was evaporated to dryness using a rotary evaporator giving a yellow oil (53.668g).

The resulting oil was dissolved in 100mL ethylacetate and was washed with brine (3x150mL). The organic phase was dried over MgSO₄ and evaporated to dryness giving a yellow oil (38.66g).

Purification was performed on a Buchi Reveleris, using hexane / EtOAc as the eluent (0-40% EtOAc). Purification was performed over a 120g silica gel column and the sample was directly used on the column. Samples were fractionated according to the ELSD detector, and checked afterwards by TLC (Heptane: EtOAc = 6:4); stained with permanganate.

The resulting diEt-GalX-octyl-mono-acetal fractions were combined, resulting in 10.17g diEt-GalX-octyl-mono-acetal (yield 39%).

The GC-MS spectrum of the diEt-GalX-octyl-mono-acetal fraction revealed closely eluting peaks with an equal molecular ion, corresponding to the different diEt-GalX-octyl-mono-acetals, and only a small peak corresponding to the diEt-GalX-octyl-di-acetals. GC-MS (EI): m/z 505 [M-CH₃]⁺.

### Example 4.2: saponification of diEt-GalX-octyl-mono-acetal

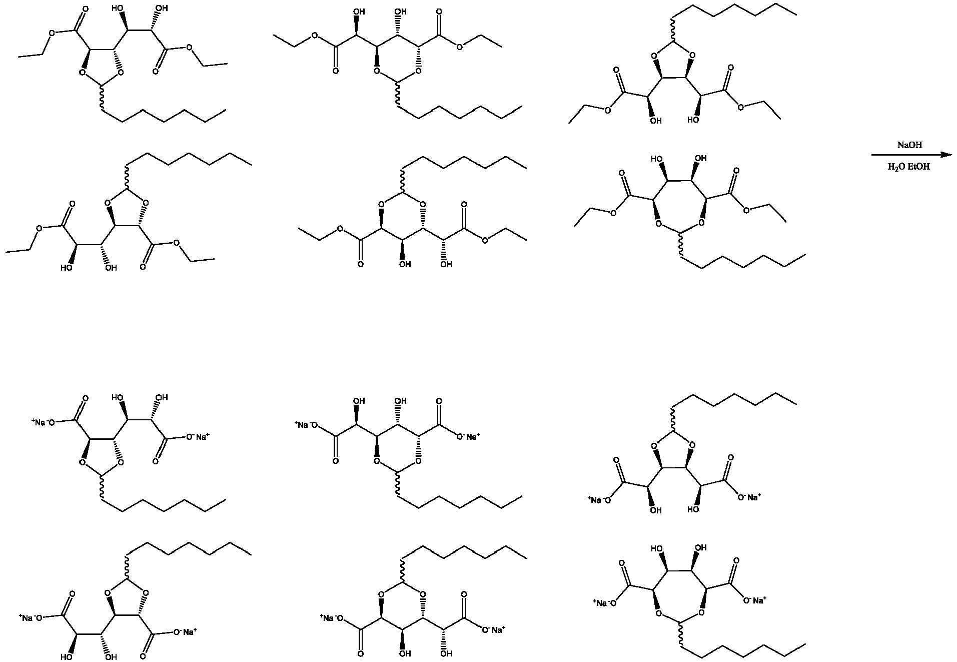

DiEt-GalX-octyl-mono-acetal (9.805 g, 0.025 mol, 1.000 eq) was dissolved in a 1:1 mixture of ethanol (34 mL) and water (34 mL) in a 250mL round bottom flask. The mixture was stirred to give an almost clear slightly yellow solution. Next, NaOH (2.062 g, 0.051 mol, 2.000 eq) was added, and the resulting mixture was heated to reflux (mantle temperature 90 °C). After 2h, the solution cooled to room temperature, and the ethanol was removed using a rotary evaporator. The resulting mixture was added dropwise into a vigorously stirred beaker containing 300 mL acetone. White solids formed during addition. Stirring was stopped after 30 min, the acetone was decanted, and 300 mL fresh acetone was added. After stirring for 30 min, the resulting white solid powder was collected by filtration over a type-3 glass filter, and dried in a vacuum oven, at 40 °C, until constant weight. The final product, GalX-octyl-mono-acetal (8.54 g), was obtained as a white powder.

### Example 5: synthesis of GaIX-butyl/decyl

### Example 5.1: synthesis of diEt-GalX-butyl/decyl-mono/di-acetal

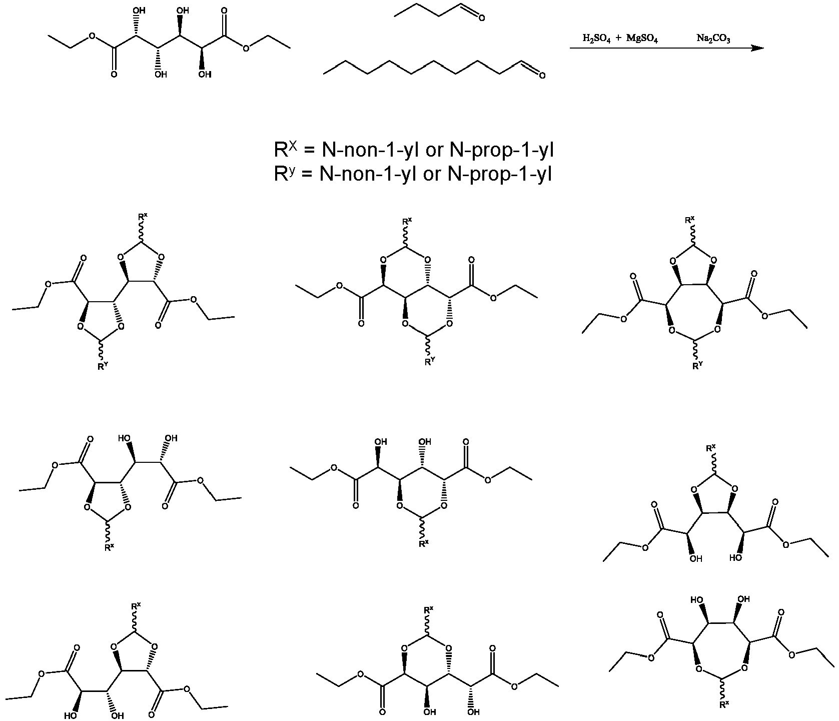

The bottle was charged with diethyl galactarate (25.000 g, 0.091 mol, 1.000 eq), decanal (33.1 mL, 0.171 mol, 1.875 eq) and butanal (16.0 mL, 0.171 mol, 1.875 eq), next under stirring MgSO₄ (12.059 g, 0.100 mol, 1.100 eq) andH₂SO₄ (18M) (7.7 mL, 0.137 mol, 1.500 eq) were added. During addition of the sulfuric acid, the white very viscous white suspension turned brown immediately, and heat development was noticed. The resulting brown suspension quickly became less viscous within a few minutes. The resulting mixture was stirred at room temperature for 60 min, after which the mixture again became more viscous. To the resulting brown suspension, solid Na₂CO₃ (14.553 g, 0.137 mol, 1.500 eq) was added to neutralize the sulfuric acid catalyst, and the suspension was stirred for 15 min. The organic layer was diluted with 200 mL chloroform, and the organic phase was extracted with sat. NaHCO₃ followed by brine to remove salts. The pH after the brine extraction was neutral. Next, the organic layer was dried over magnesium sulfate, filtered over a type-3 glass filter, and concentrated under vacuum on a rotary evaporator at 40°C. The product was obtained as 48.56 g of an light brown oil. The resulting product was analyzed by GC-MS, revealing the presence of a complex mixture of diacetals and monoacetals.

### Example 5.2: saponification of diEt-GalX-butyl/decyl-mono/di-acetal

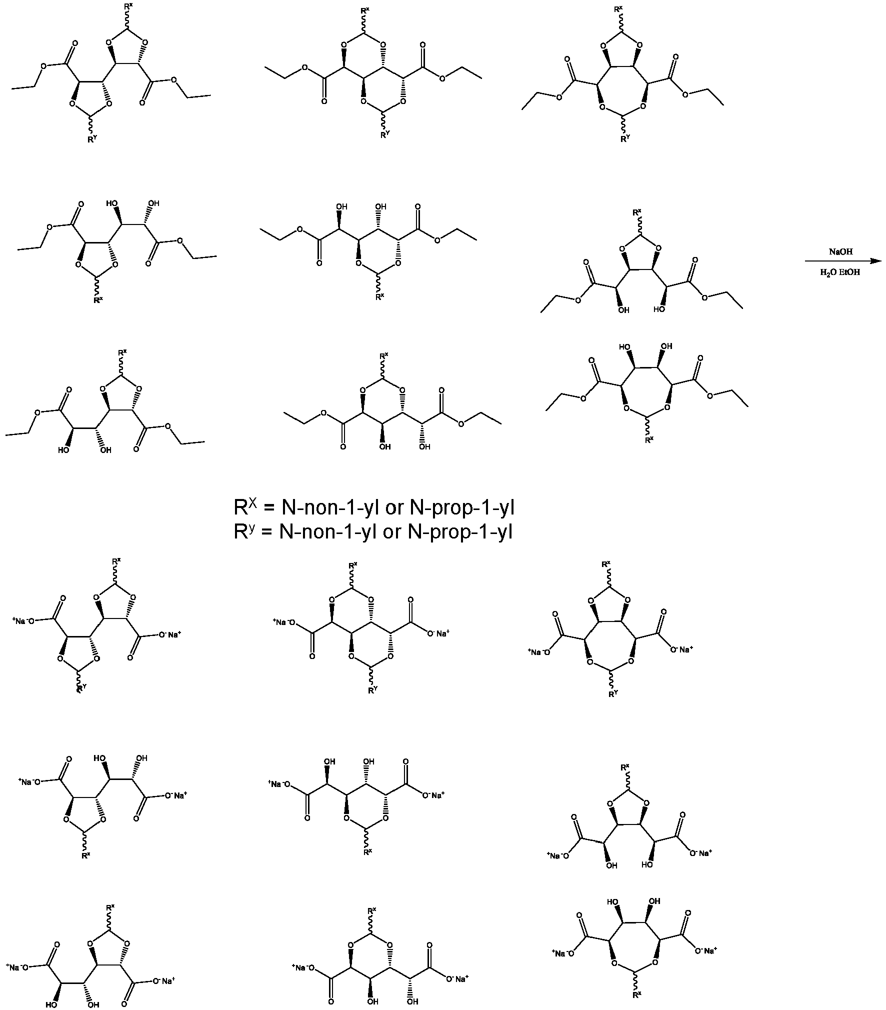

To the product of example 5.1 (48.560 g, theoretical maximum 0.091 mol, 1.000 eq), a 1:1 mixture of ethanol (60 mL) and water (60 mL) was added in a 500 mL round bottom flask. The mixture was stirred to give an almost clear yellow mixture. Next, NaOH (7.433 g, 0.182 mol, 2.000 eq) was added, and the resulting mixture was heated to reflux (mantle temperature 90°C). After 2h at reflux, a lighter yellow clear solution formed, together with a dark-yellow organic layer, and heating was stopped.

The solution cooled to room temperature, and the ethanol was removed using a rotary evaporator. The resulting mixture was added dropwise into a vigorously stirred beaker containing 600 mL acetone. Yellow oils formed during addition. Stirring was stopped, the acetone was decanted, and 400 mL fresh acetone was added. Solid yellow particles formed, and the procedure was repeated once more with 400 mL fresh acetone. The resulting yellow solid powder was collected by filtration over a type-3 glass filter, and dried in a vacuum oven, at 40 °C, until constant weight. The final product, GalX-butyl/decyl-mono/di-acetal (26.3 g), was obtained as a yellow powder.

### Example 6: synthesis of GalX-heptyl/octyl

### Example 6.1: synthesis of diEt-GalX-heptyl/octyl-mono/di-acetal

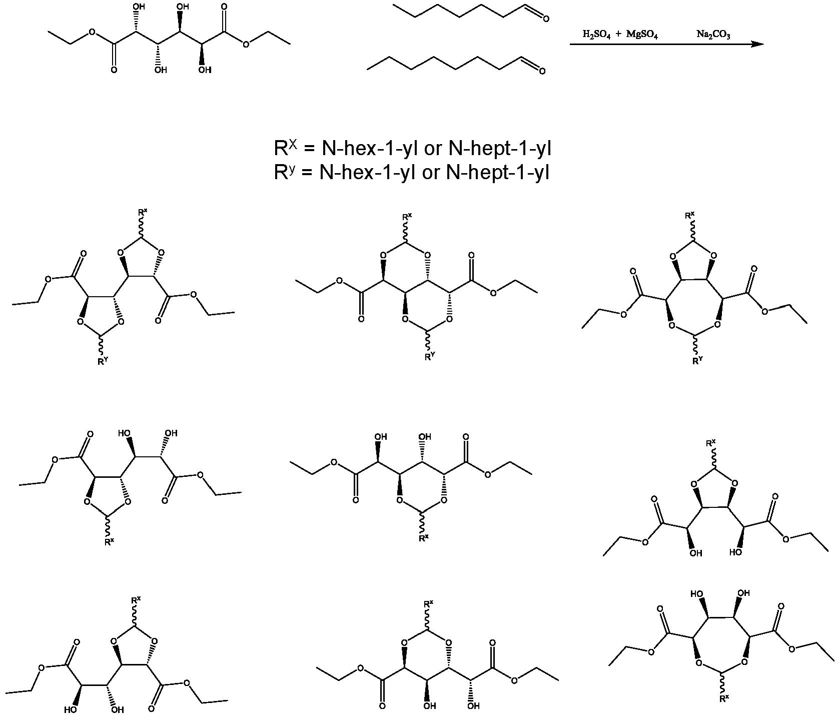

The bottle was charged with diethyl galactarate (30.000 g, 0.109 mol, 1.000 eq), heptanal (30.1 mL, 0.205 mol, 1.875 eq) and octanal (32.4 mL, 0.205 mol, 1.875 eq), next under stirring MgSO₄ (14.471 g, 0.120 mol, 1.100 eq) and H₂SO₄ (18M) (9.3 mL, 0.164 mol, 1.500 eq) were added. During addition of the sulfuric acid, the white very viscous white suspension turned brown immediately, and heat development was noticed. The resulting brown suspension quickly became less viscous within a few minutes. The resulting mixture was stirred at room temperature for 60 min, after which the mixture again became more viscous. To the resulting brown suspension, 100 mL chloroform and solid sodium Na₂CO₃ (17.464 g, 0.164 mol, 1.500 eq) was added to neutralize the sulfuric acid catalyst, and the suspension was stirred for 30 min. The organic layer was diluted with 50 mL chloroform, and the organic phase was extracted with sat. NaHCO₃ to remove salts, followed by extraction with brine. The pH after the brine extraction was neutral. Next, the organic layer was dried over magnesium sulfate, filtered over a type-3 glass filter, and concentrated under vacuum on a rotary evaporator at 40°C. The product was obtained as a thin light orange oil (60.8g, maximum purity 85%). The resulting product was analyzed by GC-MS, revealing the presence of a complex mixture of diacetals and monoacetals.

### Example 6.2: saponification of diEt-GalX-heptyl/octyl-mono/di-acetal

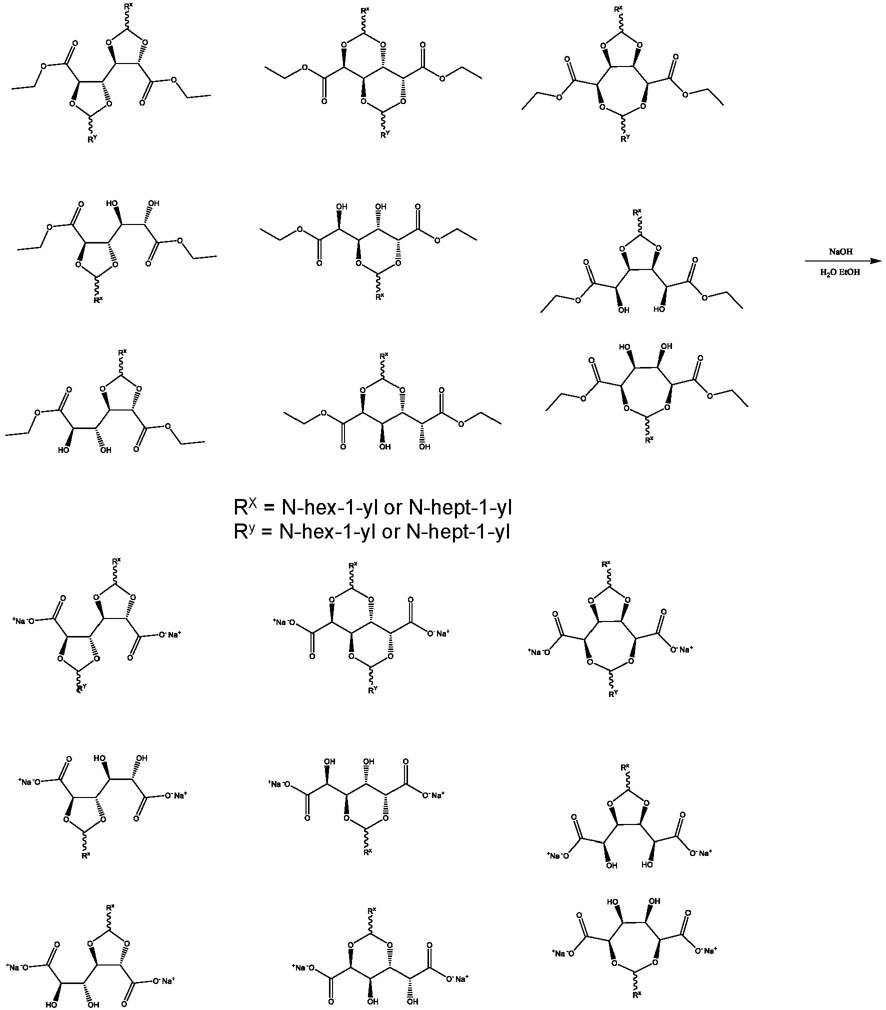

To the product of example 6.1 (60.800 g, theoretical maximum 0.109 mol, 1.000 eq), a 1:1 mixture of ethanol (60 mL) and water (60 mL) was added in a 250 mL round bottom flask. The mixture was stirred to give an almost clear yellow mixture. Next, NaOH (8.700 g, 0.213 mol, 1.949 eq) was added, and the resulting mixture was heated to reflux (mantle temperature 90°C). After 2h at reflux, a lighter yellow clear solution formed, together with a dark-yellow organic layer, and heating was stopped.

The solution cooled to room temperature, and the ethanol was removed using a rotary evaporator. The resulting mixture was extracted 2x with 100 mL diethylether to remove any side products and the organic phase was discarded. The (extracted) aqueous phase was slowly added dropwise into a vigorously stirred beaker containing 400 mL acetone. White solids formed during addition, but started to oil out. Stirring was stopped, the liquid was decanted, and 400 mL fresh acetone was added. The resulting white solid powder was collected by filtration over a type-3 glass filter, and the residual bright white powder was dried in a vacuum oven, at 40 °C, until constant weight. The final product, GalX-heptyl/octyl-mono/di-acetal (27.1 g), was obtained as a white powder.

### Example 7: effect of galactaric acid derivatives on soil removal

An experiment has been carried out to demonstrate the use of a galactaric acid derivative in a laundry detergent context.

GalX-heptyl/octyl-di-acetal was synthesized according to example 6, GalX-butyl/decyl-di-acetal was synthesized according to example 5 and GalX-octyl-mono-acetal was synthesized according to example 4.

The compositions of table 1 were then produced using the materials:

**Table 1- example compositions produced:**

| **Ingredient** | **Grams added of each ingredient** | | | |
|---|---|---|---|---|
| | **Control** | **Composition 1** | **Composition 2** | **Composition 3** |
| **Sodium - Linear Alkyl Benzenesulphonate** | 0.8 | 0.8 | 0.8 | 0.8 |
| **Sodium Laureth Sulphate** | 0.6 | 0.6 | 0.6 | 0.6 |
| **Nonionic surfactant** | 0.6 | 0.6 | 0.6 | 0.6 |
| **GaIX-heptyl/octyl-di-acetal** | **-** | 1.0 | - | - |
| **GaIX-butyl/decyl-di-acetal** | **-** | - | 1.0 | - |
| **GaIX-octyl-mono-acetal** | **-** | - | - | 1.0 |
| **Water** | 98 | 97 | 97 | 97 |

A base level of a standard Laundry Liquid (200ppm or 0.2gpL) is used as the control. This control is a 4:3:3 surfactant mix of LAS/C12-15 SLES 3EO/C12-15 7EO nonionic. The test materials are added at 100ppm/0.1gpL on top of the control formulation (i.e. to give a total ingredient loading of 300ppm or 0.3gpL). Thus a single variable test was carried to demonstrate the effect of the galactaric acid derivative materials.

### Example 7.1 - bottle wash test

The control and example compositions 1 and 2 were then tested for Indian Red soil removal. Polyester fabric swatches were pre-stained with Indian Red soil and placed into 100ml bottles.

The appropriate formulations were added to separate bottles, each containing a soiled polyester swatch. The bottles were then placed on a roller mixer at 60rpm for 45 minutes. Samples were then removed from the jars, squeezed manually to remove excess liquid and placed in 200ml deionised water for 1 minute, removed and squeezed manually again to removed excess water before being laid flat to dry overnight.

The intensity of staining was measured -optically using a X-Rite spectrometer, fitted with a 10mm aperture, readings were standardised using a piece of clean white Knitted Polyester (KPE) before reading the stain spots, according to a standard Stain Removal Index (SRI) measure. SRI measurements were calculated by reading each 'spot' before and after the cleaning process, the DE* reading from each was recorded. 4 spots were present on each cloth and 2 pots were measured for each study, therefore providing 8 measurements in each case. The SRI is equal to 100 minus the DE* reading on the X-rite for each measurement. Delta SRI was calculated from SRI post-wash minus the SRI pre-wash, therefore the greater the Delta SRI value the greater the amount of soil removed. These numbers were then averaged across all 8 data points and the standard deviation calculated to give the error bars. The results are shown in table 2.

**Table 2 - cleaning results from stain removal test**

| **Composition** | **Average difference in stain removal index** |
|---|---|
| Control | 14.4 ± 0.9 |
| Composition 1 | 20.1 ± 2.2 |
| Composition 2 | 25.1 ± 1.7 |

### Example 7.2 - lintiest washing study

Mixed loads of fabrics (cotton and polyester) were prepared and Red Pottery Clay applied. The control and compositions 2 and 3 were used to wash the fabrics and the difference in stain removal index measured for the Red Pottery Clay. The following results were obtained:

**Table 3 - cleaning results from stain removal test**

| **Composition** | **Average difference in stain removal index** |
|---|---|
| Control | 26.1 ± 0.5 |
| Composition 2 | 30.6 ± 0.6 |
| Composition 3 | 29.5 ± 1.0 |

The single variable test examples show the positive effect of the galactaric acid derivatives on soil removal.

## Claims

1. Aldaric acid derivative having a structure represented by formula (**I**): 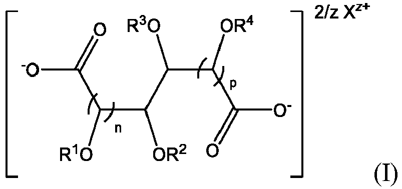 wherein:
*n* is selected from 0 and 1;
*p* is selected from 0 and 1;
when *n* is 1 and *p* is 1,
(a) R² is connected to R¹, -R¹ and -R² together form group -C(R⁵R⁶)-, R³ is connected to R⁴, and -R³ and -R⁴ together form group -C(R⁷R⁸)-; or
(b) R² is connected to R⁴, -R² and -R⁴ together form group -C(R⁵R⁶)-, R¹ is connected to R³, and -R¹ and -R³ together form group -C(R⁷R⁸)-; or
(c) R² is connected to R³, -R² and -R³ together form group -C(R⁵R⁶)-, R¹ is connected to R⁴, and -R¹ and -R⁴ together form group -C(R⁷R⁸)-; or
(d) R² is connected to R⁴, -R² and -R⁴ together form group -C(R⁵R⁶)-, and R¹ and R³ are both hydrogen; or
(e) R² is connected to R¹, -R¹ and -R² together form group -C(R⁵R⁶)-, and R³ and R⁴ are both hydrogen; or
(f) R² is connected to R³, -R² and -R³ together form group -C(R⁵R⁶)-, and R¹ and R⁴ are both hydrogen; or
(g) R¹ is connected to R⁴, -R¹ and -R⁴ together form group -C(R⁵R⁶)-, and R² and R³ are both hydrogen; or
when *n* is 0 and *p* is 1,
(h) R² is connected to R³, -R² and -R³ together form group -C(R⁵R⁶)-, and R⁴ is hydrogen; or
(i) R² is connected to R⁴, -R² and -R⁴ together form group -C(R⁵R⁶)-, and R³ is hydrogen; or
when *n* is 0 *and p* is 0,
(j) R² is connected to R³, -R² and -R³ together form group -C(R⁵R⁶)-,
wherein R⁵ is selected from the group consisting of saturated or unsaturated C₁-C₁₇ alkyl and wherein R⁶, R⁷ and R⁸ are independently selected from the group consisting of hydrogen and saturated or unsaturated C₁-C₁₇ alkyl, with the proviso that R¹, R², R³ and R⁴ together have 3 to 28 carbon atoms, and
wherein X^{*z*+} is selected from the group consisting of H⁺, metal cation M^{*z*+} with z = 1, 2 or 3, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein each R⁹ is individually chosen from the group consisting of aryl, alkyl and alkyl alcohol.

2. Aldaric acid derivative according to claim 1, wherein R¹, R², R³ and R⁴ together have 6 to 24 carbon atoms, preferably 7 to 18 carbon atoms, even more preferably 8 to 16 carbon atoms.

3. Aldaric acid derivative according to claim 1 or 2, wherein the alkyl in groups R⁵, R⁶, R⁷ and R⁸ is a linear alkyl.

4. Aldaric acid derivative according to any one of claims 1 to 3, wherein z is 1 and wherein X⁺ is selected from the group consisting of H⁺, metal cations M⁺, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein X⁺ is preferably selected from H⁺, Na⁺, K⁺, Li⁺, NH₄⁺ and ethanolammonium, wherein X⁺ more preferably is Na⁺.

5. Aldaric acid derivative according to any one of claims 1 to 4 having a structure represented by formulas (II-a), (II-b), (II-c), (II-d), (II-e), (II-f) or (II-g): 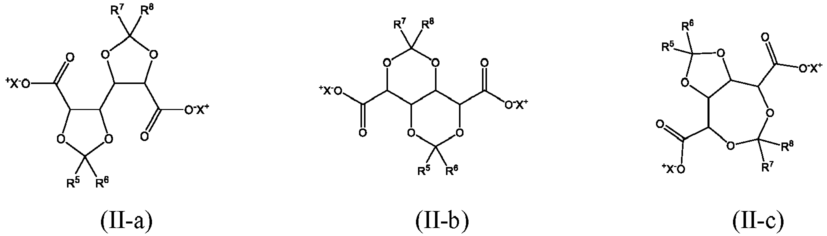 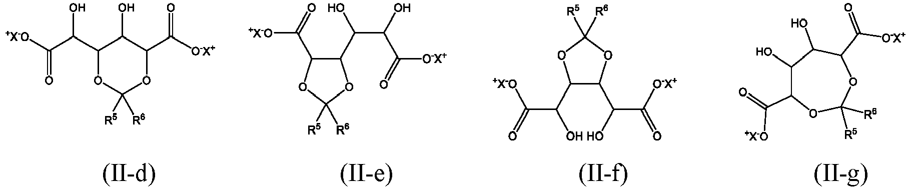 wherein z is 1 and wherein X⁺ is selected from the group consisting of H⁺, metal cations M⁺, NH₄⁺, primary ammonium cations NR⁹H₃⁺, secondary ammonium cations N(R⁹)₂H₂⁺, tertiary ammonium cations N(R⁹)₃H⁺ and quaternary ammonium cations N(R⁹)₄⁺, wherein X⁺ is preferably selected from H⁺, Na⁺, K⁺, Li⁺, NH₄⁺ and ethanolammonium, wherein X⁺ more preferably is Na⁺.

6. Aldaric acid derivative having the structure represented by formula (II-a), (II-b) or (II-c) according to claim 5, wherein R⁶ and R⁸ are hydrogen and wherein R⁵ and R⁷ are independently selected from the group consisting of saturated or unsaturated C₃-C₂₀ alkyl, preferably saturated or unsaturated C₃-C₁₂ alkyl, more preferably saturated or unsaturated C₄-C₉ alkyl.

7. Aldaric acid derivative having the structure represented by formula (II-d), (II-e), (11-f) or (II-g) according to claim 5, wherein R⁶ is hydrogen and wherein R⁵ is selected from the group consisting of saturated or unsaturated C₃-C₂₀ alkyl, preferably saturated or unsaturated C₃-C₁₆ alkyl, more preferably saturated or unsaturated C₄-C₁₂ alkyl.

8. Aldaric acid derivative having the structure represented by formula (II-d), (II-e), (11-f) or (II-g) according to claim 5, wherein R⁵ and R⁶ are independently selected from the group consisting of saturated or unsaturated C₁-C₂₀ alkyl, preferably saturated or unsaturated C₁-C₁₂ alkyl, more preferably saturated or unsaturated C₁-C₉ alkyl.

9. Aldaric acid derivative having the structure represented by formula (II-a), (II-b) or (II-c) according to claim 5, or represented by formula (II-a), (II-b) or (II-c) according to claim 6 wherein R⁵ and R⁷ are identical.

10. Aldaric acid derivative according to any one of claims 5 to 9, wherein the formulas (II-a), (II-b), (II-c), (II-d), (II-e), (II-f) or (II-g) have the galacto configuration or have the following stereochemistry:

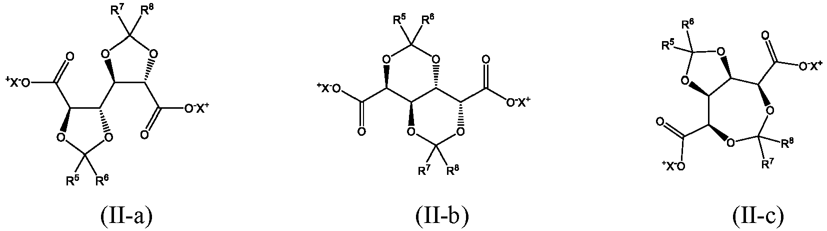

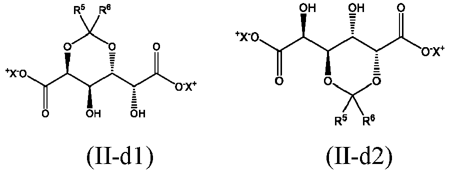

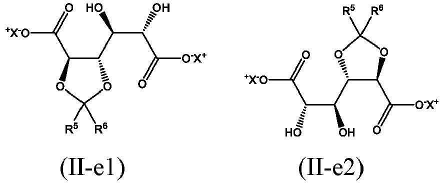

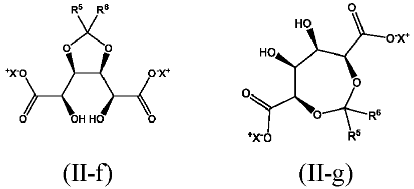

11. A composition comprising one or more aldaric acid derivatives according to any one of claims 1 to 9, wherein the one or more aldaric acid derivatives comprise more than 25 wt.% of the composition based on the total weight of the composition.

12. Method for preparing a aldaric acid derivative according to any one of claims 1-10 or a composition according to claim 11, comprising the steps of:
(i) providing an aldaric acid chosen from the group consisting of C₄, C₅ and C₆ aldaric acids;
(ii) protecting the carboxylic acid groups of an aldaric acid by esterification with an hydroxy-functionalized protecting group, preferably in the presence of an acid catalyst, preferably in a solvent;
(iii) reacting the product obtained in step (i) with an acetal, ketal, aldehyde, ketone or a mixture thereof, preferably in the presence of an acid catalyst, preferably in a solvent;
(iv) deprotecting the carboxylic acid groups of the product obtained in step (ii) via hydrolysis of the esters, preferably by saponification under alkaline conditions;
(v) optionally further conversion of the deprotected carboxylic acid groups by salt formation or salt conversion.

13. Method according to claim 12, wherein the hydroxy-functionalized protecting group is a C₁-C₄-alkyl alcohol, preferably chosen from the group consisting of methanol, ethanol, isopropyl alcohol, *n*-butyl alcohol, isobutyl alcohol and *tert*-butyl alcohol.

14. Method according to claim 12 or 13, wherein a purification step is performed:
(A) between step (ii) and step (iii); or
(B) between step (iii) and step (iv); or
(C) after step (iv); or
(D) a combination of two or more of (A)-(C).

15. Method according to any one of claims 12 to 14, wherein the acid catalyst used in step (ii) is HCl or H₂SO₄ and/or wherein the acid catalyst used in step (iii) is H₂SO₄, methanesulfonic acid, *p*-toluenesulfonic acid.

16. Method according to any one of claims 12 to 15, wherein the C₄, C₅ and C₆ aldaric acids are chosen from the group consisting of arabinaric acid, lyxaric acid, ribaric acid, xylaric acid, allaric acid, altraric acid, galactaric acid, glucaric acid, gularic acid, idaric acid, mannaric acid, talaric acid and tartaric acid.
